Europäisches Patentamt

(19) European Patent Office

Office européen des brevets

(11) Publication number: **0 227 868**
**B1**

(12) **EUROPEAN PATENT SPECIFICATION**

(45) Date of publication of the patent specification:
30.08.89

(21) Application number: **85309556.0**

(22) Date of filing: **31.12.85**

(51) Int. Cl.⁴: **C07C 49/04**, C07C 45/73,
B01J 23/64

(54) Process for production of methyl isobutyl ketone.

(43) Date of publication of application:
**08.07.87 Bulletin 87/28**

(45) Publication of the grant of the patent:
**30.08.89 Bulletin 89/35**

(84) Designated Contracting States:
**BE DE FR GB IT NL**

(56) References cited:
**FR-A- 1 412 860**
**FR-A- 2 347 098**

(73) Proprietor: **SUMITOMO CHEMICAL COMPANY, LIMITED,**
**Kitahama 4-chome 5-33, Chuo-ku Osaka 541(JP)**

(72) Inventor: **Higashio, Yasuhiko, 3-11-11, Aobadai,**
**Ichihara-shi Chiba(JP)**
Inventor: **Nakayama, Toshio, 6-13-13, Kuranamidai**
**Sodegaura-cho, Kimitsu-gun Chiba(JP)**

(74) Representative: **Moore, Anthony John et al, Gee & Co.**
**Chancery House Chancery Lane, London**
**WC2A 1QU(GB)**

## Description

The present invention relates to a process for producing methyl isobutyl ketone from acetone and hydrogen by a one-step reaction.

Methyl isobutyl ketone (hereinafter abbreviated to "MIBK") is useful as an organic solvent, and as a material for use in preparation of paints and chemical compounds such as stabilizers. MIBK is usually industrially produced from acetone and hydrogen by the following three-stage reaction:

| Acetone | condensation | Diacetone alcohol |
| | $\xrightarrow{\hspace{2cm}}$ | |
| | dehydration | Mesityl oxide |
| | $\xrightarrow{\hspace{2cm}}$ | |
| | hydrogenation | MIBK |
| | $\xrightarrow{\hspace{2cm}}$ | |

First acetone is condensed by contacting it with a solid alkaline catalyst such as barium hydroxide in the liquid phase at a temperature of 10 to 20°C and under atmospheric pressure to prepare diacetone alcohol. Then the diacetone alcohol thus prepared is separated from unreacted acetone and dehydrated by heating at 100 to 120°C in the liquid phase in the presence of an acid catalyst such as sulfuric acid or phosphoric acid to prepare mesityl oxide. Subsequently this mesityl oxide is separated and purified, and then hydrogenated in the presence of, e.g., a Raney nickel catalyst to prepare MIBK.

The above process has been widely used on a commercial scale, but has disadvantages in that the process is complicated because it needs three stages of condensation, dehydration and hydrogenation, and also separation and purification of intermediate products such as diacetone alcohol and mesityl oxide, and in that in the condensation of diacetone alcohol from acetone the conversion is as low as about 15% because the reaction is an equilibrium reaction.

For this reason there has been investigated the production of MIBK from acetone and hydrogen by a one-stage reaction. This one-stage process is very advantageous from the standpoint of equilibrium and permits an increase in the one-pass conversion of the starting material; it is thus economically advantageous over the three-stage process. Several methods of production of MIBK by a one-stage reaction have already been proposed. For example, German Patent No. 1,238,453 discloses a method of synthesizing MIBK by the use of an acid-type ion exchange resin and a palladium-carbon combination as a catalyst; Japanese Patent Publication No. 6994/74 discloses a method using a catalyst comprising zirconium phosphate having palladium deposited thereon; and Japanese Patent Publication No. 2643/71 discloses a method using a catalyst comprising an H-type zeolite having palladium deposited thereon.

These methods, however, have disadvantages in that it is not possible to increase the reaction temperature because they use resins, preparation of the catalysts is complicated, and the MIBK yield is low. Thus they are unsatisfactory for commercial use.

The present invention is intended to overcome the above problems of the conventional one-stage processes for production of MIBK; and has as its aim the provision of a process for the production of MIBK in high yield by a simplified procedure.

It has been found that MIBK can be produced in high yield by a one-stage reaction by contacting acetone and hydrogen in the presence of a catalyst comprising a niobic acid and palladium.

Niobic acid is a solid otherwise called hydrated niobium oxide, and its properties are described in "Third Solid Acid Process Conference Preprint", pp. 1-4, Nov. 11, 1983 (Catalysis Society of Japan). It is reported that niobic acid possesses acidic properties and has activity in the hydration and esterification of ethylene, but its catalytic action in other reactions is virtually unknown.

The catalyst used in the present invention can take various forms: for example, palladium deposited on niobic acid, or a physical mixture of niobic acid and various palladium catalysts such as palladium-carbon, palladium-alumina or palladium black. In addition, palladium may be deposited on a mixed carrier of niobic acid and alumina or silica. Where a catalyst comprising niobic acid having palladium deposited thereon is used, the amount of palladium deposited or the mixing ratio of palladium is preferably 0.01 to 5.0 wt% and more preferably 0.02 to 1.0 wt%.

The process of the present invention can be carried out in various ways such as so-called fixed bed flow reaction in which a catalyst comprising niobic acid and palladium is packed in an adiabatic or isothermic reactor, and acetone and hydrogen are passed therethrough; and a suspension reaction in which a catalyst comprising niobic acid and palladium is suspended in acetone and hydrogen is blown therethrough. In the case of the fixed bed flow reaction, acetone and hydrogen may be reacted in either the liquid phase or gas phase. In some cases, a reaction system such as trickle phase may be employed. In the case of the suspension reaction, the reaction may be carried out either batchwise or continuously. In the fixed bed flow reaction, it is preferred to use a catalyst comprising niobic acid having palladium deposited hereon, and in the suspension reaction, it is preferred to use a mixture of niobic acid and a palladium catalyst.

In the practice of the process of the present invention, the reaction temperature is usually 80 to 250°C and preferably 120 to 200°C. If the reaction temperature is less than 80°C, the rate of reaction is low. On the other hand, if it is more than 250°C, the amounts of highly condensed products of acetone formed are increased.

The reaction pressure is usually from atmospheric pressure to $50 \cdot 0.1 \times 10^5$ Pa (50 atms). Although a preferred reaction pressure varies with the reaction temperature employed, it is from $10 \cdot 0.1$ to $30 \cdot 0.5 \cdot 10^5$ Pa (10 to 30 atms).

The catalyst of the present invention has high activity and stability, and good selectivity. Moreover, the catalyst has a long service life. Thus the present invention permits the production of MIBK in high yield and in a stable manner for a long period of time.

The present invention is described below in greater detail be reference to the following Examples although it is not intended to be limited thereto.

## EXAMPLE 1

A 200-millimeter autoclave provided with a magnetic stirrer was charged with 100 ml of acetone, and then 2 g of niobic acid (a powder having a water content of 7 wt%, produced by CBMM Co.) and 1 g of palladium-alumina (a powder having a palladium content of 0.1 wt%, produced by Sumitomo Aluminum Co., Ltd) were introduced in the autoclave. The autoclave was heated to 160°C and hydrogen was introduced in the autoclave, and the reaction was carried out while stirring in such a manner that the pressure in the autoclave was $19 \cdot 61 \times 10^5$ Pa (20 kg/cm²). Hydrogen was continuously supplemented for compensation of consumed hydrogen so that the total pressure was always maintained at $19 \cdot 61 \times 10^5$ Pa (20 kg/cm²). After reacting for 2 hours, the autoclave was cooled, and the reaction mixture was taken out of the autoclave and separated from hydrogen and the catalyst. The reaction mixture was analyzed by gas chromotography. The results were as follows:

| | |
|---|---|
| Acetone conversion: | 49.3% |
| MIBK selectivity: | 92.3% |
| IPA (isopropanol) selectivity: | 0.8% |
| DIBK (diisobutyl ketone) selectivity: | 3.8% |

## COMPARATIVE EXAMPLE

The same procedure as in Example 1 was repeated with the exception that only the palladium-alumina was used in an amount of 3 g. The results were as follows:

| | |
|---|---|
| Acetone conversion: | 3.8% |
| MIBK selectivity: | 62.3% |
| IPA selectivity: | 35.8% |
| DIBK selectivity: | 0.6% |

It can be seen from the above results that the palladium-alumina catalyst alone had almost no activity in production of MIBK from acetone by a one-stage reaction.

## EXAMPLES 2 AND 3

The same procedure as in Example 1 was repeated with the exception that each of the catalysts shown in Table 2 was used. The results are shown in Table 1.

Table 1

| Example No. | Catalyst | Acetone Conversion (%) | Selectivity (%) | | |
|---|---|---|---|---|---|
| | | | MIBK | IPA | DIBK |
| 2 | Niobic acid: 2 g 5% Pd-Carbon: 1 g | 43.1 | 86.3 | 7.4 | 2.1 |
| 3 | Niobic acid with 0.1% Pd deposited: 3 g | 47.3 | 93.5 | 0.4 | 3.4 |

EXAMPLES 4 TO 7

The same procedure as in Example 1 was repeated with the exception that the reaction conditions were changed as shown in Table 2. The results are shown in Table 2.

Table 2

| Example No. | Reaction Temperature (°C) | Reaction Pressure Pa (kg/cm$^2$) | Acetone Conversion (%) | Selectivity (%) | | |
|---|---|---|---|---|---|---|
| | | | | MIBK | IPA | DIBK |
| 4 | 140 | $19.6 \times 10^5$ (20) | 38.4 | 93.8 | 1.3 | 2.6 |
| 5 | 180 | $29.42 \times 10^5$ (30) | 56.2 | 91.3 | 1.1 | 4.3 |
| 6 | 160 | $29.42 \times 10^5$ (30) | 50.1 | 92.5 | 1.4 | 3.9 |
| 7 | 160 | $39.23 \times 10^5$ (40) | 51.2 | 91.8 | 2.1 | 3.7 |

EXAMPLE 8

Niobic acid (a cylindrical form having a water content of 7 wt%, produced by CBMM Co.) was soaked in an aqueous solution of palladium chloride, reduced with hydrazine, and then calcined at 300°C. The amount of palladium deposited was 0.1 wt%.

Then 100 ml of the catalyst thus prepared was packed in a vertically aligned reactor having an inner diameter of 28 mm. Under conditions of temperature of 160°C and pressure of $19.61 \times 10^5$ Pa (20 kg/cm$^2$), acetone and hydrogen were introduced in the reactor at rates of 158 g/hr (LHSV=2) and 265 Nml/min, respectively, and reacted. The results are shown in Table 3.

Table 3

| Reaction Time (hr) | Acetone Conversion (%) | Selectivity (%) | | |
|---|---|---|---|---|
| | | MIBK | IPA | DIBK |
| 10 | 41.8 | 93.5 | 0.3 | 3.6 |
| 100 | 40.3 | 93.6 | 0.2 | 4.2 |
| 500 | 41.2 | 92.8 | 0.2 | 3.8 |

EXAMPLE 9

The same procedure as in Example 6 was repeated with the exception that the reaction pressure was changed to $9.81 \times 10^5$ Pa (10 kg/cm$^2$) and that acetone was reacted in the gas phase The results (after 10 hours) are shown below.

| Acetone conversion: | 43.8% |
|---|---|
| MIBK selectivity: | 90.1% |
| IPA selectivity: | 0.1% |
| DIBK selectivity: | 5.8% |

## Claims

1. A process for producing methyl isobutyl ketone in a one-stage reaction which comprises contacting acetone and hydrogen in the presence of a catalyst comprising palladium, characterised in that the catalyst also comprises niobic acid.

2. A process as claimed in Claim 1, wherein the palladium is deposited on the niobic acid.

3. A process as claimed in Claim 2, wherein the amount of palladium is from 0.01 to 5.0 wt%.

4. A process as claimed in Claim 1, wherein the niobic acid is in the form of a physical mixture with a palladium catalyst.

5. A catalyst comprising palladium and niobic acid.

## Patentansprüche

1. Verfahren zur Herstellung von Methylisobutylketon in einem Einstufenverfahren, wobei Aceton und Wasserstoff in Anwesenheit eines palladiumhaltigen Katalysators in Berührung gebracht werden, dadurch gekennzeichnet, daß der Katalysator auch Niobsäure enthält.

2. Verfahren nach Anspruch 1, wobei das Palladium auf der Niobsäure abgeschieden ist.

3. Verfahren nach Anspruch 2, wobei die Menge des Palladiums von 0,01 bis 5,0 Gew.-% beträgt.

4. Verfahren nach Anspruch 1, wobei die Niobsäure in Form eines physikalischen Gemisches mit einem Palladiumkatalysator vorliegt.

5. Katalysator enthaltend Palladium und Niobsäure.

## Revendications

1. Un procédé pour la production de méthylisobutylcétone dans une réaction en un stade qui consiste à mettre en contact l'acétone et l'hydrogène en présence d'un catalyseur comprenant du palladium, caractérisé en ce que le catalyseur comprend également de l'acide niobique.

2. Un procédé selon la revendication 1, dans lequel le palladium est déposé sur l'acide niobique.

3. Un procédé selon la revendication 2, dans lequel la quantité de palladium est de 0,01 à 5,0% en poids.

4. Un procédé selon la revendication 1, dans lequel l'acide niobique est sous la forme d'un mélange physique avec un catalyseur au palladium.

5. Un catalyseur comprenant du palladium et de l'acide niobique.